# EUROPÄISCHE PATENTSCHRIFT

(11) **EP 3 179 853 B1**
(45) Veröffentlichungstag und Bekanntmachung des Hinweises auf die Patenterteilung: **03.04.2019**
(21) Anmeldenummer: 15753923.0
(22) Anmeldetag: 12.08.2015
(51) Int. Cl.: A01N 25/02, A01N 25/22, A01N 37/02, A01N 37/40, A01N 39/02, A01N 39/04, A01N 41/10, A01N 43/40, A01N 43/42, A01N 43/78, A01N 47/34, A01P 13/00

(54) **ORGANISCHE AMMONIUMSALZE VON ANIONISCHEN PESTIZIDEN**
ORGANIC AMMONIUM SALTS OF ANIONIC PESTICIDES
SELS ORGANIQUES D'AMMONIUM DE PESTICIDES ANIONIQUES

(30) Priorität: 13.08.2014 DE 102014012022
(43) Veröffentlichungstag der Anmeldung: 21.06.2017
(60) Teilanmeldung: 19157188.4
(73) Patentinhaber: Clariant International Ltd, 4132 Muttenz (CH)
(72) Erfinder: KLUG, Peter, 63762 Grossostheim (DE); BAUR, Peter, 86938 Schondorf (DE); BAUER, Martin, 60329 Frankfurt am Main (DE)
(74) Vertreter: Paczkowski, Marcus
(86) Internationale Anmeldenummer: PCT/EP2015/068602
(87) Internationale Veröffentlichungsnummer: WO 2016/023963

(56) Entgegenhaltungen:
- EP-A2- 0 412 849
- WO-A2-2007/063335
- DE-A1- 1 542 964
- DE-A1-102007 002 924
- DE-B1- 1 493 954
- JP-A- 2010 037 252
- DATABASE WPI Week 201463 Thomson Scientific, London, GB; AN 2014-P64140 XP002745337, -& RU 2 525 911 C1 (TETS G V) 20. August 2014 (2014-08-20)

## Beschreibung

Die Erfindung betrifft organische Ammoniumsalze von anionischen Pestiziden, ein Verfahren zu deren Herstellung, agrochemische Zusammensetzungen, welche diese Salze enthalten, sowie Verfahren zur Bekämpfung von Schadorganismen unter Verwendung der genannten Salze und Zusammensetzungen.

Pestizide (vor allem Herbizide, Fungizide und Insektizide) sind chemische Substanzen synthetisch hergestellt oder natürlichen Ursprungs, die in Pflanzenzellen, -gewebe oder in parasitäre Organismen in oder auf der Pflanze eindringen und diese schädigen und/oder zerstören. Den größten Anteil an Pestiziden stellen Herbizide dar. Pestizide werden üblicherweise in Form von flüssigen oder festen konzentrierten Zubereitungen (Formulierungen) in der Landwirtschaft eingesetzt. Diese erleichtern dem Anwender so die Handhabung oder sorgen für eine höhere Wirksamkeit des Wirkstoffs. Die Formulierungen werden üblicherweise vor dem Einsatz mit Wasser verdünnt und anschließend durch Sprühapplikation ausgebracht.

Wasserlösliche Konzentrate (Soluble Liquids, abgekürzt mit SL) sind eine wichtige Form der Pestizidzubereitungen. Sie spielen insbesondere bei Herbiziden eine große Rolle, wobei die Pestizide oftmals als wasserlösliche Salze, die durch Neutralisation der Säureform der Herbizide mit geeigneten Basen in ihre Alkali- oder Ammoniumsalze überführt werden, eingesetzt werden. Unter Umständen ist ein zweiter nicht wasserlöslicher Wirkstoff in der Pestizidzubereitung enthalten. Dann handelt es sich um ein Suspensionskonzentrat (SC), auch wenn in der wässrigen Phase ein Wirkstoff gelöst ist.

Eine besonders wichtige Rolle spielen die wasserlöslichen Salze von Herbiziden, beispielsweise des Glyphosats, Glufosinats oder der Auxin-Herbizide wie Clodinafop, 2,4-D, MCPA oder Quinclorac. Sie werden vorzugsweise als Alkalimetallsalz oder in Form verschiedener Ammoniumsalze bzw. als Gemisch dieser Salze meistens als wässrige Formulierungen verwendet.

Bei der Anwendung von Pestiziden ist es von Vorteil, wenn diese eine geringe Flüchtigkeit aufweisen, da eine hohe Flüchtigkeit insbesondere mit einem verstärkten Verwehen (Drift) kleinerer Spritztröpfchen unter 150mm Tropfendurchmesser der Pestizide mit hohen Verlusten und Eintrag in die Nichtzielvegetation beim Sprühen verbunden ist. Ein solches Verwehen ist aus ökologischen und ökonomischen Gründen unerwünscht, da unbeabsichtigte Schäden verursacht werden könnten und die Wirkung der Pestizide auf die Zielorganismen herabgesetzt wird.

Um diesen Effekt zu vermeiden, ist es bekannt, flüchtige Pestizide, die in Form freier Säuren vorliegen, als Salze einzusetzen.

US 4,405,531 und WO 97/24931 offenbaren verschiedene organische Salze von Glyphosate mit Di- und Polyaminen.

In US 5,221,791 sind Aminoalkylpyrrolidon-Salze von Pestiziden wie Dicamba beschrieben.

EP-A 0 375 624 offenbart schwerflüchtige Salze von Pestiziden mit verschiedenen Polyaminen.

In der EP-A 0 183 384 sind schwerflüchtige Salze von Dicamba mit Aminoalkoholen beschrieben.

DE-A 15 42 964 beschreibt Herbizide auf Basis von Glucaminsalzen substituierter Cyanophenolate.

EP-A 0 412 849 offenbart Azolverbindungen mit pestizider Wirkung.

In EP-A 0 614 881 sind Verfahren zur Herstellung von tertiären Dialkylpolyhydroxyaminen beschrieben.

Obwohl mit den bekannten Systemen bereits gute Ergebnisse erzielt werden, bleibt doch ein breiter Raum für Verbesserungen, insbesondere im Hinblick auf eine Erhöhung der Wasserlöslichkeit der Wirkstoffe im Konzentrat, eine geringere Volatilität der Wirkstoffe und eine verbesserte Wirksamkeit durch bessere Aufnahme.

Es wurde nun gefunden, dass sich die Salze von Mono- und Dialkylglucaminen mit anionischen Pestiziden in besonderer Weise zum Einsatz als wässrige Spritzapplikationen eignen.

Gegenstand der Erfindung ist daher ein organisches Ammoniumsalz der Formel (I), 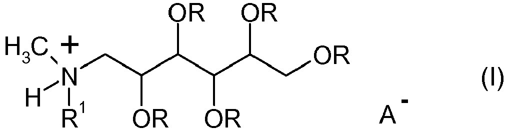 wobei die Symbole folgende Bedeutungen haben:
- A⁻: ist ein anionisches Pestizid,
- R¹: CH₃ und
- R: ist H,
dadurch gekennzeichnet, dass A ausgewählt ist aus der Gruppe bestehend aus: Aminocyclopyrachlor, Aminopyralid, Benazolin, Clopyralid, 2,4-D, 2,4-DB, 2,4,5-T, Dicamba, Dichlorprop, Dichlorprop-P, Diflufenzopyr, Fluroxypyr, MCPA, MCPB, Mecoprop, Mecoprop-P, Picloram, Quinclorac, Quinmerac, Tricamba, Triclopyr, Indol-3-essigsäure, 1-Naphthylessigsäure, 2-Naphthylessigsäure und Salicylsäure.

Weiterhin Gegenstand der Erfindung ist ein Verfahren zur Herstellung eines erfindungsgemäßen Ammoniumsalzes, wobei man die protonierte Form eines anionischen Herbizids mit einem Glucamin der Formel (II) umsetzt, 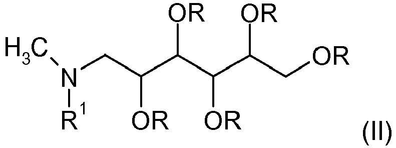 worin die Symbole die in der Formel (I) angegebenen Bedeutungen haben.

Weiterhin Gegenstand der Erfindung ist die Verwendung der erfindungsgemäßen Ammoniumsalze als Pestizide.

Ebenso Gegenstand der Erfindung ist eine Pestizidzusammensetzung, enthaltend
a) ein oder mehrere erfindungsgemäße Ammoniumsalze (I) und
b) einen oder mehrere Formulierungshilfsstoffe.

Ebenso Gegenstand der Erfindung ist ein Verfahren zur Bekämpfung von Schadorganismen, wobei man den Schadorganismus oder dessen Lebensraum mit einem erfindungsgemäßen Ammoniumsalz (I) oder einer erfindungsgemäßen Pestizidzusammensetzung in Kontakt bringt.

Weiterhin Gegenstand der Erfindung ist die Verwendung eines erfindungsgemäßen Ammoniumsalzes zur Verminderung der Flüchtigkeit des als Salz vorliegenden anionischen Pestizids.

Die erfindungsgemäßen Ammoniumsalze (I) eignen sich in hervorragender Weise zum Einsatz als wässrige Spritzapplikationen und zeigen insbesondere eine gute Wasserlöslichkeit und verminderte Flüchtigkeit bei nicht erhöhter Drift in der Anwendung.

Das Kation der erfindungsgemäßen Ammoniumsalze (I) entsteht durch Protonierung eines Glucamins (II), 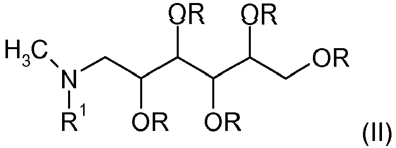 wobei die Symbole folgende Bedeutungen haben:
- R¹: ist CH₃ und
- R: ist H

Der Pentahydroxyhexylrest in den Alkylglucaminen der Formel (I) und (II) verfügt über verschiedene chirale Zentren, so dass jeweils mehrere Stereoisomere existieren können. Üblicherweise werden die Alkylglucamine der Formel (II) aus natürlich vorkommenden Zuckern, wie der D-Glucose hergestellt, grundsätzlich ist aber auch die Verwendung anderer natürlicher oder synthetischer Hexosen oder anderer C₆-Bausteine möglich, so dass unterschiedliche Stereoisomere der Formeln (I) und (II) resultieren können.

Die Herstellung der Alkylglucamine der Formel (II) ist hinlänglich vorbeschrieben und dem Fachmann bekannt. Sie erfolgt beispielsweise wie in der EP-A 1 676 831 beschrieben durch reduktive Alkylierung von N-Alkylpolyhydroxylaminen mit Aldehyden oder Ketonen in Gegenwart von Wasserstoff und eines Übergangsmetallkatalysators.

Dimethylglucamin kann beispielsweise gemäß EP-A 0 614 881 hergestellt werden. Dimethylglucamin ist als 50 % wässrige Lösung einsetzbar. Dimethylglucamin ist als tertiäres Amin wenig anfällig für die Bildung von Nitrosaminen. Die Alkylglucamine der Formel (II) basieren bevorzugt auf nachwachsenden Rohstoffen und zeichnen sich durch ein vorteilhaftes toxikologisches und ökologisches Profil aus.

Die Gruppe (A)⁻ in der Formel (I) steht für ein anionisches Pestizid wie oben angegeben.

Das anionische Pestizid ist bevorzugt die konjugierte Base einer pestiziden Brønsted Säure, die einem pKₐ-Wert von 1,5 bis 7, bevorzugt 2 bis 6 und besonders bevorzugt 2,5 bis 5,5 aufweist.

Bevorzugt weist die konjugierte Base des anionischen Pestizids mindestens eine Carbonsäure-, Thiocarbonsäure-, Sulfonsäure-, Thiosulfonsäure-, Sulfinsäure-, Phosphonsäure-, Sulfonylharnstoff-, Sulfonicarbamat-, Phenol-, Hydrobenzonitril-, Ketoenol- und/oder Triketongruppe auf. Bevorzugt sind Carbonsäure- und Phosphonsäuregruppen.

Vorzugsweise genannt seien als Pestizide anionische Herbizide, Pflanzenwuchsregulatoren. Insektizide, Akarizide, Fungizide, Bakterizide, Nematizide, Pflanzennährstoffe und Repellents. Bevorzugt sind anionische Herbizide, Wachstumsregulatoren und Insektizide, insbesondere Herbizide.

Erfindungsgemäß sind die folgenden anionischen Pestizide aus der Gruppe der Herbizide: Aminocyclopyrachlor, Aminopyralid, Benazolin, Clopyralid, 2,4-D, 2,4-DB, 2,4,5-T, Dicamba, Dichlorprop, Dichlorprop-P, Diflufenzopyr, Fluroxypyr, MCPA, MCPB, Mecoprop, Mecoprop-P, Picloram, Quinclorac, Quinmerac, Tricamba und Triclopyr.

Erfindungsgemäß sind weiterhin die folgenden anionischen Pestizide aus der Gruppe der Wachstumsregulatoren: Indol-3-essigsäure, 1-Naphthylessigsäure, 2-Naphthylessigsäure und Salicylsäure.

Bevorzugt als anionische Pestizide sind Clopyralid, 2,4-D (2,4-Dichlorphenoxyessigsäure), Dicamba, MCPA, Quinclorac, Quinmerac, Salicylsäure und Triclopyr.

Besonders bevorzugt sind Clopyralid, 2,4-D, Dicamba, MCPA und Triclopyr.

Insbesondere bevorzugt sind Clopyralid, 2,4-D und Dicamba.

Bevorzugt, besonders bevorzugt und insbesondere bevorzugt sind auch die Dimethylglucammoniumsalze der bevorzugten, besonders bevorzugt beziehungsweise insbesondere bevorzugten anionischen Pestizide A⁻.

Die genannten Pestizide sind bekannt und kommerziell erhältlich. Sie sind beispielsweise beschrieben in Weed Research 26 (1986) 441 445 oder "The Pesticide Manual", 16th edition, The British Crop Protection Council and the Royal Soc. of Chemistry, 2012 und der dort zitierten Literatur.

Die erfindungsgemäßen Ammoniumsalze (I) zeigen im Wesentlichen dasselbe Wirkspektrum wie die entsprechenden Pestizide A und können somit gegen dieselben, dem Fachmann bekannten Zielorganismen eingesetzt werden.

Mit den erfindungsgemäßen Ammoniumsalzen (I) lassen sich erfindungsgemäße Pestizidzusammensetzungen, insbesondere wässrige Herbizid-Zusammensetzungen herstellen, mit ausgezeichneter Wirkung und ausgezeichneten anwendungstechnischen Eigenschaften, wie der verringerten Flüchtigkeit ohne veränderte Drift.

Die Pestizidzusammensetzungen enthalten
a) ein oder mehrere erfindungsgemäße Ammoniumsalze (I), wie oben beschrieben, und
b) einen oder mehrere Formulierungshilfsstoffe.

In einer bevorzugten Ausführungsform der Erfindung beträgt die Menge des einen oder der mehreren Ammoniumsalze (I) a) in den erfindungsgemäßen Zusammensetzungen mehr als 100 g/l, bevorzugt mehr als 200 g/l und besonders bevorzugt mehr als 300 g/l. Diese Mengenangaben beziehen sich auf das Gesamtgewicht der erfindungsgemäßen Pestizidzusammensetzung und auf die Menge an freier Säure (d.h. der protonierten Form), dem sogenannten Säureäquivalent ("acid equivalent", a.e.) der anionischen Pestizide A.

Bei den Formulierungshilfsstoffen (b) handelt es sich beispielsweise um Lösungsmittel, Tenside, Entschäumer, funktionelle Polymere, Adjuvants, Frostschutzmittel, Konservierungsmittel, Farbstoffe und/oder Ammoniumsalze. Bevorzugt als Lösungsmittel ist Wasser. Bevorzugt ist auch der Einsatz von einem oder mehreren Cosolventien. Bei den Cosolventien kann es sich um ein einziges Lösemittel oder ein Gemisch zweier oder mehrerer Lösemittel handeln. Dazu eignen sich alle polaren Lösemittel, die mit der wässrigen Pestizidzusammensetzung kompatibel sind und eine homogene Phase bilden. Geeignete Cosolventien sind beispielsweise einwertige Alkohole, wie Methanol, Ethanol, Propanole, Butanole, Benzylalkohol oder weitere mehrwertige Alkohole wie Ethylenglykol, Diethylenglykol, Propylenglykol, Dipropylenglykol, Glycerin oder Polyglykole wie Polyethylen-, Polypropylen- oder gemischte Polyalkylenglykole (PAGs). Weitere geeignete Lösemittel sind Ether wie beispielsweise Propylenglykolmono- oder dimethylether, Dipropylenglykolmono- oder dimethylether, Amide wie beispielsweise N-Methyl- oder N-Ethylpyrrolidon, Milchsäure-, Capryl- oder Decansäuredimethylamid.

Bei den Tensiden kann es sich generell um alle mit der Zusammensetzung kompatiblen nichtionischen, amphoteren, kationischen oder anionische Tenside handeln.

Beispiele für nicht-ionische Tenside sind Glucamide, insbesondere wie in der WO 2014/067663 beschrieben, Ethoxylate und Alkoxylate von längerkettigen aliphastischen oder aromatischen Alkoholen, Fettaminethoxylate, längerkettige Etheraminalkoxylate, (gegebenenfalls ethoxylierte) Sorbitanester, Alkylpolyglycoside. Geeignete amphotere Tenside sind u.a. langkettige Alkyldimethylbetaine oder Alkyldimethylaminoxide, oder Alkyldimethylaminamidopropylaminoxide. Unter den anionischen Tensiden sind beispielsweise Ethersulfate von ethoxylierten Fettalkoholen, Umsetzungsprodukte von (gegebenenfalls ethoxylierten) langkettigen Alkoholen mit Phosphorsäurederivaten geeignet. Unter dem Begriff "langkettig" werden lineare oder verzweigte Kohlenwasserstoffketten mit mindestens 6 und maximal 22 Kohlenstoffatomen verstanden.

Als Entschäumer eignen sich Fettsäurealkylesteralkoxylate, Organopolysiloxane wie Polydimethylsiloxane und deren Gemische mit mikrofeiner, gegebenenfalls silanierter Kieselsäure; Perfluoralkylphosphonate und -phosphinate, Paraffine, Wachse und Mikrokristallinwachse und deren Gemische mit silanierter Kieselsäure. Vorteilhaft sind auch Gemische verschiedener Schauminhibitoren, beispielsweise solche aus Silikonöl, Paraffinöl und/oder Wachsen. Bevorzugt sind die in der deutschen Patentanmeldung DE 10 2014 208 244.7 beschriebenen Entschäumer.

Bei den funktionellen Polymeren, die in der erfindungsgemäßen Pestizid-Zusammensetzung enthalten sein können, handelt es sich hochmolekulare Verbindungen synthetischen oder natürlichen Ursprungs mit einer Molmasse von größer als 10.000. Die funktionellen Polymere können beispielsweise als Antidrift-Agent wirken oder die Regenfestigkeit steigern.

In einer weiteren Ausführungsform der Erfindung enthalten die erfindungsgemäßen Pestizidzusammensetzungen ein oder mehrere Adjuvantien, wie sie bekanntermaßen in wässrigen Pestizidzusammensetzungen verwendet werden können.

Bevorzugt sind dies Fettaminethoxylate, Etheraminethoxylate, Alkylbetaine oder Amidoalkylbetaine, Aminoxide oder Amidoalkylaminoxide, Alkylpolylglycoside oder Copolymere aus Glycerin, Kokosfettsäure und Phthalsäure.

Diese Adjuvantien sind aus der Literatur bekannt und beispielsweise in der WO 2009/029561 beschrieben.

Weiterhin bevorzugt als Adjuvantien sind Glucamide, insbesondere wie in der WO 2014/067663 beschrieben.

Ebenso bevorzugt als Adjuvantien sind driftreduzierende Polyglycerinester wie in der WO 2014/063818 beschrieben, welche ein oder mehrere Copolymere A) enthalten, wobei die Copolymere eine oder mehrere Struktureinheiten, hervorgegangen aus
a) 19,9 bis 75,9 Gew.-% Glycerin
b) 0,1 bis 30 Gew.-% mindestens einer Dicarbonsäure und
c) 24 bis 80 Gew.-% mindestens einer Monocarbonsäure gemäß Formel (III),

   R²-COOH (III)

   wobei R² (C₅-C₂₉)-Alkyl; (C₇-C₂₉)-Alkenyl; Phenyl oder Naphthyl darstellt, enthalten.

Als Konservierungsmittel können organische Säuren und ihre Ester, beispielsweise Ascorbinsäure, Ascorbinpalmitat, Sorbat, Benzoesäure, Methyl- und Propyl-4-hydroxybenzoat, Propionate, Phenol, beispielsweise 2-Phenylphenat, 1,2 Benzisothiazolin-3-on, Formaldehyd, schwefelige Säure und deren Salze eingesetzt werden.

Als Frostschutzmittel eignen sich beispielsweise Ethylenglykol, Propylenglykol, Glycerin und Harnstoff

Als Ammoniumsalze eignen sich wasserlösliche Ammoniumsalze wie Ammoniumsulfat, Ammoniumnitrat, Ammoniumnitratharnstoff, Ammoniumphosphat, Ammoniumcitrat, Ammoniumthiosulfat und/oder Ammoniumchlorid, bevorzugt Ammoniumsulfat, Ammoniumnitrat, Ammoniumcitrat und/oder Ammoniumnitratharnstoff, besonders bevorzugt Ammoniumsulfat.

Neben den Ammoniumsalzen (I) a) und Formulierungshilfsstoffen b) können die Pestizidzusammensetzungen weitere Pestizide c) enthalten.

Im Folgenden werden Beispiele für Pestizide c) genannt, die Kombinationspartner der Ammoniumsalze (I) bilden können. Soweit es sich um anionische Pestizide handelt, können diese natürlich auch als Ammoniumsalze der Formel (I) eingesetzt werden.

Als Beispiele für Herbizide seien genannt:
Wirkstoffe, die auf einer Inhibition von beispielsweise Acetolaktat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441 445 oder "The Pesticide Manual", 16th edition, The British Crop Protection Council and the Royal Soc. of Chemistry, 2012 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acibenzolar, Acibenzolar-S-methyl, Aclonifen, Alachlor, Allidochlor, Alloxydim, Alloxydim-sodium, Ametryn, Amicarbazone, Amidochlor, Amidosulfuron, Aminocyclopyrachlorkalium, Aminocyclopyrachlormethyl, Ammoniumsulfamat, Ancymidol, Anilofos, Atrazine, Aviglycin, Azafenidin, Azimsulfuron, Aziprotryn, Beflubutamid, Benazolinethyl, Bencarbazone, Benfluralin, Benfuresate, Bensulide, Benzfendizone, Benzobicyclon, Benzofenap, Benzofluor, Benzoylprop, Benzyladenin, Bromobutide, Bromofenoxim, Bromuron, Buminafos, Busoxinone, Butachlor, Butafenacil, Butamifos, Butenachlor, Butralin, Butroxydim, Butylate, Cafenstrole, Carbaryl, Carbetamide, Carvone, Chlorcholinchlorid, Chlomethoxyfen, Chlorbromuron, Chlorbufam, Chlorfenac, Chlorfenacnatrium, Chlorfenprop, Chlorflurenol-methyl, Chloridazon, Chlorimuronethyl, Chlormequatchlorid, Chlornitrofen, 4-Chlorophenoxyacetic acid, Chlorophthalim, Chlorpropham, Chlorotoluron, Chlorsulfuron, Cinidon, Cinidonethyl, Cinmethylin, Cinosulfuron, Clethodim, Clodinafoppropargyl, Clofencet, Clomazone, Clomeprop, Cloprop, Cloransulam, Cloransulammethyl, Cloxyfonac, Cumyluron, Cyanamide, Cyanazine, Cyclanilide, Cycloate, Cyclosulfamuron, Cycloxydim, Cycluron, Cyperquat, Cyprazine, Cyprazole, Cytokinine, Daimuron/Dymron, Daminozide, Dazomet, n-Decanol, Desmedipham, Desmetryn, Detosyl-Pyrazolate (DTP), Diallate, Diaminozid, Dichlobenil, Diclosulam, Diethatyl, Diethatylethyl, Difenoxuron, Diflufenican, Diflufenzopyrnatrium, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Diisopropylnaphthalene, Dipropetryn, Dithiopyr, Diuron, DNOC, Eglinazineethyl, EPTC, Esprocarb, Ethalfluralin, Ethametsulfuron, Ethametsulfuronmethyl, Ethylnaphthylacetat, Ethephon, Ethidimuron, Ethiozin, Ethofumesate, Ethoxyfen, Ethoxyfenethyl, Ethoxysulfuron, Etobenzanid, F-5331, d.h. N-[2-Chlor-4-fluor-5-[4-(3-fluorpropyl)-4,5-dihydro-5-oxo-1H-tetrazol-1-yl]-phenyl]-ethansulfonamid, F-7967, d. h. 3-[7-Chlor-5-fluor-2-(trifluormethyl)-1H-benzimidazol-4-yl]-1-methyl-6-(trifluormethyl) pyrimidin-2,4(1H,3H)-dion, Fenoxasulfone, Fentrazamide, Fenuron, Flazasulfuron, Fluazolate, Flucarbazonesodium, Flucetosulfuron, Fluchloralin, Flufenacet (Thiafluamide), Flumetralin, Flumetsulam, Flumioxazin, Flumipropyn, Fluometuron, Fluorodifen, Flupoxam, Flupropacil, Flupyrsulfuron, Flupyrsulfuronmethylsodium, Fluridone, Flurochloridone, Flurprimidol, Flurtamone, Fluthiacetmethyl, Fluthiamide, Forchlorfenuron, Furyloxyfen, Gibberellinsäure, H-9201, d. h. O-(2,4-Dimethyl-6-nitrophenyl)-O-ethylisopropylphosphor amidothioat, Halosafen, Halosulfuronmethyl, Hexazinone, HW-02, d. h. 1-(Dimethoxyphosphoryl)-ethyl-(2,4-dichlorphenoxy)acetat, Imazosulfuron, Inabenfide, Indanofan, Indaziflam, lodosulfuron, lodosulfuronmethyl-natrium, Iofensulfuron, Iofensulfuronnatrium, Ioxynil, Ipfencarbazone, Isocarbamid, Isopropalin, Isoproturon, Isouron, Isoxaben, Isoxachlortole, Isoxaflutole, KUH-043, d. h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Karbutilate, Ketospiradox, Lenacil, Linuron, Maleinsäurehydrazid, Mefenacet, Mefluidide, Mepiquat-chlorid, Methabenzthiazuron, Metam, Metamitron, Metazachlor, Metazasulfuron, Methazole, Methiopyrsulfuron, Methiozolin, Methoxyphenone, Methyldymron, 1-Methylcyclopropen, Methylisothiocyanat, Metobenzuron, Metobromuron, Metolachlor, S-Metolachlor, Metosulam, Metoxuron, Metribuzin, Metsulfuron, Metsulfuronmethyl, Molinate, Monalide, Monocarbamide, Monocarbamidedihydrogensulfat, Monolinuron, Monosulfuron, Monosulfuronester, Monuron, MT-128, d. h. 6-Chlor-N-[(2E)-3-chlorprop-2-en-1-yl]-5-methyl-N-phenylpyridazin-3-amin, MT-5950, d. h. N-[3-Chlor-4-(1-methylethyl)-phenyl]-2-methylpentanamid, NGGC-011, Naproanilide, Napropamide, NC-310, d.h. 4-(2,4-Dichlorobenzoyl)-1-methyl-5-benzyloxypyrazole, Neburon, Nipyraclofen, Nitralin, Nitrofen, Nitroguaiacolate, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquatdichlorid, Pendimethalin, Pendralin, Penoxsulam, Pentanochlor, Pentoxazone, Perfluidone, Pethoxamid, Phenisopham, Phenmedipham, Phenmediphamethyl, Picolinafen, Pinoxaden, Piperophos, Pirifenop, Pirifenopbutyl, Pretilachlor, Probenazole, Profluazol, Procyazine, Prodiamine, Prifluraline, Profoxydim, Prohexadionecalcium, Prohydrojasmone, Prometon, Prometryn, Propachlor, Propanil, Propazine, Propham, Propisochlor, Propyrisulfuron, Propyzamide, Prosulfalin, Prosulfocarb, Prosulfuron, Prynachlor, Pyraclonil, Pyrasulfotole, Pyrazolynate (Pyrazolate), Pyrazosulfuron, Pyrazosulfuronethyl, Pyrazoxyfen, Pyribambenz, Pyribambenz-isopropyl, Pyribambenzpropyl, Pyribenzoxim, Pyributicarb, Pyridafol, Pyridate, Pyriftalid, Pyrimisulfan, Pyroxasulfone, Pyroxsulam, Quinoclamine, Rimsulfuron, Saflufenacil, Secbumeton, Sethoxydim, Siduron, Simazine, Simetryn, SN-106279, d. h. Methyl-(2R)-2-({7-[2-chlor-4-(trifluormethyl)phenoxy]-2-naphthyl}oxy)propanoat, Sulfallate (CDEC), Sulfentrazone, Sulfo-sulfuron, SW-065, SYN-523, SYP-249, d.h. 1-Ethoxy-3-methyl-1-oxobut-3-en-2-yl-5-[2-chlor-4-(trifluormethyl)phenoxy]-2-nitrobenzoat, SYP-300, d.h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, Tebutam, Tebuthiuron, Tecnazene, Tefuryltrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazonemethyl, Thifensulfuron, Thifensulfuronmethyl, Thiobencarb, Tiocarbazil, Tralkoxydim, Triafamone, Triallate, Triasulfuron, Triaziflam, Triazofenamide, Tribenuron, Tribenuronmethyl, Tribufos, Tridiphane, Trietazine, Trifloxysulfuron, Trifloxysulfuronnatrium, Trifluralin, Triflusulfuron, Triflusulfuronmethyl, Trimeturon, Trinexapacethyl, Tritosulfuron, Tsitodef, Uniconazole, Uniconazole-P, Vernolate, ZJ-0862, d. h. 3,4-Dichlor-N-{2-[(4,6-dimethoxypyrimidin-2-yl)oxy]benzyl}anilin.

Als Beispiele für Pflanzenwuchsregulatoren seien ferner natürliche Pflanzenhormone wie Ester der Salicylsäure, Kinetin und Brassinosteroide genannt.

Weiter seien Substanzen genannt, die als Pflanzenwuchsregulatoren und/oder Pflanzenstärkungsmittel wirken können, um den Einfluss von Stressfaktoren wie Hitze, Kälte, Trockenheit, Salz, Sauerstoffmangel bzw.- Überschwemmung auf das Pflanzenwachstum zu mindern. Hier seien beispielhaft genannt Glycinbetain (Betain), Cholin, Kaliumphosphat oder andere Phosphatsalze, sowie Silikate.

Als Beispiele für Pflanzennährstoffe seien übliche anorganische oder organische Dünger zur Versorgung von Pflanzen mit Makro- und/oder Mikronährstoffen genannt.

Als Beispiele für Fungizide seien genannt:
(1) Inhibitoren der Ergosterol-Biosynthese, wie beispielsweise Aldimorph, Azaconazol, Bitertanol, Bromuconazol, Cyproconazol, Diclobutrazol, Difenoconazol, Diniconazol, Diniconazol-M, Dode-morph, Dodemorph Acetat, Epoxiconazol, Etaconazol, Fenarimol, Fenbuconazol, Fenhexamid, Fenpropidin, Fenpropimorph, Fluquinconazol, Flurprimidol, Flusilazol, Flutriafol, Furconazol, Furconazol-Cis, Hexaconazol, Imazalil, Imazalil Sulfat, Imibenconazol, Ipconazol, Metconazol, Myclobutanil, Naftifin, Nuarimol, Oxpoconazol, Paclobutrazol, Pefurazoat, Penconazol, Piperalin, Prochloraz, Propiconazol, Prothioconazol, Pyributicarb, Pyrifenox, Quinconazol, Simeconazol, Spiroxamin, Tebuconazol, Terbinafin, Tetraconazol, Triadimefon, Triadimenol, Tridemorph, Triflumizol, Triforin, Triticonazol, Uniconazol, Uniconazol-p, Viniconazol, Voriconazol, 1-(4-Chlorphenyl)-2-(1H-1,2,4-triazol-1-yl)cycloheptanol, Methyl-1-(2,2-dimethyl-2,3-dihydro-1H-inden-1-yl)-1H-imidazol-5-carboxylat, N'-{5-(Difluormethyl)-2-methyl-4-[3-(trimethylsilyl)propoxy]phenyl}-N-ethyl-N-methylimidoformamid, N-Ethyl-N-methyl-N'-{2-methyl-5-(trifluormethyl)-4-[3-(trimethylsilyl)propoxy]phenyl}imidoformamid und O-[1-(4-Methoxyphenoxy)-3,3-dimethylbutan-2-yl]-1H-imidazol-1-carbothioat.
(2) Inhibitoren der Respiration (Atmungsketten-Inhibitoren), wie beispielsweise Bixafen, Boscalid, Carboxin, Diflumetorim, Fenfuram, Fluopyram, Flutolanil, Fluxapyroxad, Furametpyr, Furmecyclox, Isopyrazam Mischung des synepimeren Razemates 1RS,4SR,9RS und des antiempimeren Razemates 1RS,4SR,9SR, Isopyrazam (anti-epimeres Razemat), Isopyrazam (anti-epimeres Enantiomer 1R,4S,9S), Isopyrazam (anti-epimeres Enantiomer 1S,4R,9R), Isopyrazam (synepimeres Razemat 1RS,4SR,9RS), Isopyrazam (synepimeres Enantiomer 1R,4S,9R), Iso-pyrazam (syn-epimeres Enantiomer 1S,4R,9S), Mepronil, Oxycarboxin, Penflufen, Penthiopyrad, Sedaxane, Thifluzamid, 1-Methyl-N-[2-(1,1,2,2-tetrafluorethoxy)phenyl]-3-(trifluormethyl)-1H-pyrazol-4-carboxamid, 3-(Difluormethyl)-1-methyl-N-[2-(1,1,2,2-tetrafluorethoxy)phenyl]-1H-pyrazol-4-carboxamid, 3-(Difluormethyl)-N-[4-fluor-2-(1,1,2,3,3,3-hexafluorpropoxy)phenyl]-1-methyl-1H-pyrazol-4-carboxamid, N-[1-(2,4-Dichlorphenyl)-1-methoxypropan-2-yl]-3-(difluormethyl)-1-methyl-1H-pyrazol-4-carboxamid, 5,8-Difluor-N-[2-(2-fluor-4-{[4-(trifluormethyl)pyridin-2-yl]oxy}phenyl)ethyl]quinazolin-4-amin, N-[9-(Dichlormethylen)-1,2,3,4-tetrahydro-1,4-methanonaphthalen-5-yl]-3-(difluormethyl)-1-methyl-1H-pyrazol-4-carboxamid, N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid.
(3) Inhibitoren der Respiration (Atmungsketten-Inhibitoren) am Komplex III der Atumungskette, wie beispielsweise Ametoctradin, Amisulbrom, Azoxystrobin, Cyazofamid, Coumethoxystrobin, Coumoxystrobin, Dimoxystrobin, Enestroburin, Famoxadon, Fenamidon, Fenoxystrobin, Fluoxastrobin, Kresoxim-Methyl, Metominostrobin, Orysastrobin, Picoxystrobin, Pyraclostrobin, Pyrametostrobin, Pyraoxystrobin, Pyribencarb, Triclopyricarb, Trifloxystrobin, (2E)-2-(2-{[6-(3-Chlor-2-methylphenoxy)-5-fluorpyrimidin-4-yl]oxy}phenyl)-2-(methoxyimino)-N-methylethanamid, (2E)-2-(Methoxyimino)-N-methyl-2-(2-{[({(1E)-1-[3-(trifluormethyl)phenyl]ethyliden}amino)oxy]methyl}phenyl)ethanamid, (2E)-2-(Methoxyimino)-N-methyl-2-{2-[(E)-({1-[3-(trifluormethyl)phenyl]ethoxy}imino)methyl]phenyl}ethanamid, (2E)-2-{2-[({[(1E)-1-(3-{[(E)-1-Fluor-2-phenylethenyl]oxy}phenyl)ethyliden]-amino}oxy)methyl]phenyl}-2-(methoxyimino)-N-methylethanamid, (2E)-2-{2-[({[(2E,3E)-4-(2,6-Dichlorphenyl)but-3-en-2-yliden]amino}oxy)methyl]phenyl}-2-(methoxyimino)-N-methylethanamid, 2-Chlor-N-(1,1,3-trimethyl-2,3-dihydro-1H-inden-4-yl)pyridin-3-carboxamid, 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, Methyl-(2E)-2-{2-[({cyclopropyl[(4-methoxyphenyl)imino]methyl}sulfanyl)methyl]phenyl}-3-methoxyprop-2-enoat, N-(3-Ethyl-3,5,5-trimethylcyclohexyl)-3-(formylamino)-2-hydroxybenzamid, 2-{2-[(2,5-Dimethylphenoxy)methyl]phenyl}-2-methoxy-N-methylacetamid und (2R)-2-{2-[(2,5-Dimethylphenoxy)methyl]phenyl}-2-methoxy-N-methylacetamid.
(4) Inhibitoren der Mitose und Zellteilung, wie beispielsweise Benomyl, Carbendazim, Chlorfe-nazol, Diethofencarb, Ethaboxam, Fluopicolid, Fuberidazol, Pencycuron, Thiabendazol, Thi-ophanat-Methyl, Thiophanat, Zoxamid, 5-Chlor-7-(4-methylpiperidin-1-yl)-6-(2,4,6-trifluorphenyl)[1,2,4]triazolo[1,5-a]pyrimidin und 3-Chlor-5-(6-chlorpyridin-3-yl)-6-methyl-4-(2,4,6-trifluorphenyl)pyridazin.
(5) Verbindungen mit Multisite-Aktivität, wie beispielsweise Bordeauxmischung, Captafol, Captan, Chlorothalonil, Kupferzubereitungen wie Kupferhydroxid, Kupfernaphthenat, Kupferoxid, Kupferoxychlorid, Kupfersulfat, Dichlofluanid, Dithianon, Dodine, Dodine freie Base, Ferbam, Fluorofolpet, Folpet, Guazatin, Guazatinacetat, Iminoctadin, Iminoctadinalbesilat, Iminoctadintriacetat, Mankupfer, Mancozeb, Maneb, Metiram, Zinkmetiram, Kupfer-Oxin, Propamidin, Propineb, Schwefel und Schwefelzubereitungen wie beispielsweise Calciumpolysulfid, Thiram, Tolylfluanid, Zineb und Ziram.
(6) Resistenzinduktoren, wie beispielsweise Acibenzolar-S-Methyl, Isotianil, Probenazol und Tiadinil.
(7) Inhibitoren der Aminosäure- und Protein-Biosynthese, wie beispielsweise Andoprim, Blasti-cidin-S, Cyprodinil, Kasugamycin, Kasugamycin Hydrochlorid Hydrat, Mepanipyrim, Pyrimethanil und 3-(5-Fluor-3,3,4,4-tetramethyl-3,4-dihydroisoquinolin-1-yl)quinolin.
(8) Inhibitoren der ATP Produktion, wie beispielsweise Fentin Acetat, Fentin Chlorid, Fentin Hydroxid und Silthiofam.
(9) Inhibitoren der Zellwandsynthese, wie beispielsweise Benthiavalicarb, Dimethomorph, Flu-morph, Iprovalicarb, Mandipropamid, Validamycin A und Valifenalat.
(10) Inhibitoren der Lipid- und Membran-Synthese, wie beispielsweise Biphenyl, Chloroneb, Dicloran, Edifenphos, Etridiazol, lodocarb, Iprobenfos, Isoprothiolan, Propamocarb, Propamocarb Hydrochlorid, Prothiocarb, Pyrazophos, Quintozen, Tecnazene und Tolclofos-Methyl.
(11) Inhibitoren der Melanin-Biosynthese, wie beispielsweise Carpropamid, Diclocymet, Fenoxanil, Fthalid, Pyroquilon, Tricyclazol und 2,2,2-Trifluorethyl {3-methyl-1-[(4-methylbenzoyl)amino]butan-2-yl}carbamat.
(12) Inhibitoren der Nukleinsäuresynthese, wie beispielsweise Benalaxyl, Benalaxyl-M (Kirala-xyl), Bupirimat, Clozylacon, Dimethirimol, Ethirimol, Furalaxyl, Hymexazol, Metalaxyl, Metalaxyl-M (Mefenoxam), Ofurace, Oxadixyl und Oxolinsäure.
(13) Inhibitoren der Signaltransduktion, wie beispielsweise Chlozolinat, Fenpiclonil, Fludioxonil, Iprodion, Procymidon, Quinoxyfen und Vinclozolin.
(14) Entkoppler, wie beispielsweise Binapacryl, Dinocap, Ferimzon, Fluazinam und Meptyldinocap.
(15) Weitere Verbindungen, wie beispielsweise Benthiazol, Bethoxazin, Capsimycin, Carvon, Chinomethionat, Pyriofenon (Chlazafenon), Cufraneb, Cyflufenamid, Cymoxanil, Cyprosulfa-mide, Dazomet, Debacarb, Dichlorophen, Diclomezin, Difenzoquat, Difenzoquat Methylsulphat, Diphenylamin, Ecomat, Fenpyrazamin, Flumetover, Fluoromid, Flusulfamid, Flutianil, Fosetyl-Aluminium, Fosetyl-Calcium, Fosetyl-Natrium, Hexachlorbenzol, Irumamycin, Methasulfocarb, Methylisothiocyanat, Metrafenon, Mildiomycin, Natamycin, Nickel Dimethyldithiocarbamat, Nitrothal-Isopropyl, Octhilinone, Oxamocarb, Oxyfenthiin, Pentachlorphenol und dessen Salze, Phenothrin, Phosphorsäure und deren Salze, Propamocarb-Fosetylat, Propanosin-Natrium, Proquinazid, Pyrimorph, (2E)-3-(4-Tert-butylphenyl)-3-(2-chlorpyridin-4-yl)-1-(morpholin-4-yl)prop-2-en-1-on, (2Z)-3-(4-Tert-butylphenyl)-3-(2-chlorpyridin-4-yl)-1-(morpholin-4-yl)prop-2-en-1-on, Pyrrolnitrin, Tebufloquin, Tecloftalam, Tolnifanid, Triazoxid, Trichlamid, Zarilamid, (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat, 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-Methoxyphenoxy)-3,3-dimethylbutan-2-yl-1H-imidazol-1-carboxylat, 2,3,5,6-Tetrachlor-4-(methylsulfonyl)pyridin, 2,3-Dibutyl-6-chlorthieno[2,3-d]pyrimidin-4(3H)-on, 2,6-Dimethyl-1H,5H-[1,4]dithiino[2,3-c:5,6-c']dipyrrol-1,3,5,7(2H,6H)-tetron, 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, 2-Butoxy-6-iod-3-propyl-4H-chromen-4-on, 2-Chlor-5-[2-chlor-1-(2,6-difluor-4-methoxyphenyl)-4-methyl-1H-imidazol-5-yl]pyridin, 2-Phenylphenol und dessen Salze, 3-(4,4,5-Trifluor-3,3-dimethyl-3,4-dihydroisoquinolin-1-yl)quinolin, 3,4,5-Trichlorpyridin-2,6-dicarbonitril, 3-[5-(4-Chlorphenyl)-2,3-dimethyl-1,2-oxazolidin-3-yl]pyridin, 3-Chlor-5-(4-chlorphenyl)-4-(2,6-difluorphenyl)-6-methylpyridazin, 4-(4-Chlorphenyl)-5-(2,6-difluorphenyl)-3,6-dimethylpyridazin, 5-Amino-1,3,4-thiadiazol-2-thiol, 5-Chlor-N'-phenyl-N'-(prop-2-in-1-yl)thiophen-2-sulfonohydrazid, 5-Fluor-2-[(4-fluorbenzyl)oxy]pyrimidin-4-amin, 5-Fluor-2-[(4-methylbenzyl)oxy]pyrimidin-4-amin, 5-Methyl-6-octyl[1,2,4]triazolo[1,5-a]pyrimidin-7-amin, Ethyl-(2Z)-3-amino-2-cyan-3-phenylprop-2-enoat, N'-(4-{[3-(4-Chlorbenzyl)-1,2,4-thiadiazol-5-yl]oxy}-2,5-dimethylphenyl)-N-ethyl-N-methylimidoformamid, N-(4-Chlorbenzyl)-3-[3-methoxy-4-(prop-2-in-1-yloxy)phenyl]propanamid, N-[(4-Chlorphenyl)(cyan)methyl]-3-[3-methoxy-4-(prop-2-in-1-yloxy)phenyl]propanamid, N-[(5-Brom-3-chlorpyridin-2-yl)methyl]-2,4-dichlorpyridin-3-carboxamid, N-[1-(5-Brom-3-chlorpyridin-2-yl)ethyl]-2,4-dichlorpyridin-3-carboxamid, N-[1-(5-Brom-3-chlorpyridin-2-yl)ethyl]-2-fluor-4-iodpyridin-3-carboxamid, N-{(E)-[(Cyclopropylmethoxy)imino][6-(difluormethoxy)-2,3-difluorphenyl]methyl}-2-phenylacetamid, N-{(Z)-[(Cyclopropyl-methoxy)¬imino][6-(difluormethoxy)-2,3-difluorphenyl]methyl}-2-phenylacetamid, N'-{4-[(3-Tert-butyl-4-cyano-1,2-thiazol-5-yl)oxy]-2-chlor-5-methylphenyl}-N-ethyl-N-methylimidoformamid, N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid, N-Methyl-2-(1-{[5-methyl-3-(trifluormethyl)-1H-pyrazol-1-yl]acetyl}¬piperidin-4-yl)-N-[(1R)-1,2,3,4-tetrahydronaphthalen-1-yl]-1,3-thiazol-4-carboxamid, N-Methyl-2-(1-{[5-methyl-3-(trifluormethyl)-1H-pyrazol-1-yl]acetyl}piperidin-4-yl)-N-[(1S)-1,2,3,4-tetrahydronaph¬thalen-1-yl]-1,3-thiazol-4-carboxamid, Pentyl-{6-[({[(1-methyl-1H-tetrazol-5-yl)(phenyl)methyliden]amino}oxy)methyl]pyridin-2-yl}carbamat, Phenazin-1-carbonsäure, Chinolin-8-ol, Chinolin-8-olsulfat(2:1) und Tert-butyl {6-[({[(1-methyl-1H-tetrazol-5-yl)(phenyl)methylen]amino}oxy)methyl]pyridin-2-yl}carbamat.
(16) Weitere Verbindungen, wie beispielsweise 1-Methyl-3-(trifluormethyl)-N-[2'-(trifluormethyl)biphenyl-2-yl]-1H-pyrazol-4-carboxamid, N-(4'-Chlorbiphenyl-2-yl)-3-(difluormethyl)-1-methyl-1H-pyrazol-4-carboxamid, N-(2',4'-Dichlorbiphenyl-2-yl)-3-(difluormethyl)-1-methyl-1H-pyrazol-4-carboxamid, 3-(Difluormethyl)-1-methyl-N-[4'-(trifluormethyl)biphenyl-2-yl]-1H-pyrazol-4-carboxamid, N-(2',5'-Difluorbiphenyl-2-yl)-1-methyl-3-(trifluormethyl)-1H-pyrazol-4-carboxamid, 3-(Difluormethyl)-1-methyl-N-[4'-(prop-1-in-1-yl)biphenyl-2-yl]-1H-pyrazol-4-carboxamid, 5-Fluor-1,3-dimethyl-N-[4'-(prop-1-in-1-yl)biphenyl-2-yl]-1H-pyrazol-4-carboxamid, 2-Chlor-N-[4'-(prop-1-in-1-yl)biphenyl-2-yl]pyridin-3-carboxamid, 3-(Difluormethyl)-N-[4'-(3,3-dimethylbut-1-in-1-yl)biphenyl-2-yl]-1-methyl-1H-pyrazol-4-carboxamid, N-[4'-(3,3-Dimethylbut-1-in-1-yl)biphenyl-2-yl]-5-fluor-1,3-dimethyl-1H-pyrazol-4-carboxamid, 3-(Difluormethyl)-N-(4'-ethinylbiphenyl-2-yl)-1-methyl-1H-pyrazol-4-carboxamid, N-(4'-Ethinylbiphenyl-2-yl)-5-fluor-1,3-dimethyl-1H-pyrazol-4-carboxamid, 2-Chlor-N-(4'-ethinylbiphenyl-2-yl)pyridin-3-carboxamid, 2-Chlor-N-[4'-(3,3-dimethylbut-1-in-1-yl)biphenyl-2-yl]pyridin-3-carboxamid, 4-(Difluormethyl)-2-methyl-N-[4'-(trifluormethyl)biphenyl-2-yl]-1,3-thiazol-5-carboxamid, 5-Fluor-N-[4'-(3-hydroxy-3-methylbut-1-in-1-yl)biphenyl-2-yl]-1,3-dime¬thyl-1H-pyrazol-4-carboxamid, 2-Chlor-N-[4'-(3-hydroxy-3-methylbut-1-in-1-yl)biphenyl-2-yl]pyridin-3-carboxamid, 3-(Difluormethyl)-N-[4'-(3-methoxy-3-methylbut-1-in-1-yl)biphenyl-2-yl]-1-methyl-1H-pyrazol-4-carboxamid, 5-Fluor-N-[4'-(3-methoxy-3-methylbut-1-in-1-yl)biphenyl-2-yl]-1,3-dimethyl-1H-pyrazol-4-carboxamid, 2-Chlor-N-[4'-(3-methoxy-3-methylbut-1-in-1-yl)biphenyl-2-yl]pyridin-3-carboxamid, (5-Brom-2-methoxy-4-methylpyridin-3-yl)(2,3,4-trimethoxy-6-methylphenyl)methanon, N-[2-(4-{[3-(4-Chlorphenyl)prop-2-in-1-yl]oxy}-3-methoxyphenyl)ethyl]-N2-(methylsulfonyl)valinamid, 4-Oxo-4-[(2-phenylethyl)amino]butansäure und But-3-yn-1-yl {6-[({[(Z)-(1-methyl-1H-tetrazol-5-yl)(phenyl)methylen]amino}oxy)methyl]pyridin-2-yl}carbamat.

Alle genannten Pestizide (1) bis (16) können, wenn sie aufgrund ihrer funktionellen Gruppen dazu imstande sind, gegebenenfalls mit geeigneten Basen oder Säuren Salze bilden.

Als Beispiele für Bakterizide seien genannt:
Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

Als Beispiele für Insektizide, Akarizide und Nematizide seien genannt:
(1) Acetylcholinesterase (AChE) Inhibitoren, wie beispielsweise
   Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder
   Organophosphate, z.B. Acephate, Azamethiphos, Azinphosethyl, Azinphosmethyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphosmethyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise
   Cyclodienorganochlorine, z.B. Chlordane und Endosulfan; oder
   Phenylpyrazole (Fiprole), z.B. Ethiprole und Fipronil.
(3) Natrium-Kanal-Modulatoren / Spannungsabhängige Natrium-Kanal-Blocker, wie beispielsweise
   Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)-Isomere)], Tralomethrin und Transfluthrin; oder DDT; oder Methoxychlor.
(4) Nikotinerge Acetylcholin-Rezeptor (nAChR) Agonisten, wie beispielsweise Neonikotinoide, z.B. Acetamiprid, Clothianidin, Dinotefuran, Imidacloprid, Nitenpyram, Thiacloprid und Thiamethoxam; oder
   Nikotin.
(5) Nikotinerge Acetylcholin-Rezeptor (nAChR) allosterische Aktivatoren, wie beispielsweise
   Spinosine, z.B. Spinetoram und Spinosad.
(6) Chlorid-Kanal-Aktivatoren, wie beispielsweise Avermectine/Milbemycine, z.B. Abamectin, Emamectin-benzoat, Lepimectin und Milbemectin.
(7) Juvenilhormon-Imitatoren, wie beispielsweise Juvenilhormon-Analoge, z.B. Hydroprene, Kinoprene und Methoprene; oder Fenoxycarb; oder Pyriproxyfen.
(8) Wirkstoffe mit unbekannten oder nicht spezifischen Wirkmechanismen, wie beispielsweise
   Alkylhalide, z.B. Methylbromid und andere Alkylhalide; oder
   Chloropicrin; oder Sulfurylfluorid; oder Borax; oder Brechweinstein.
(9) Selektive Fraßhemmer, z.B. Pymetrozine; oder Flonicamid.
(10) Milbenwachstumsinhibitoren, z.B. Clofentezine, Hexythiazox und Diflovidazin; oder
   Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron; oder
   Organozinnverbindungen, z.B. Azocyclotin, Cyhexatin und Fenbutatin-oxid; oder Propargite; oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, Dipteran, wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; oder Acequinocyl; oder Fluacrypyrim.
(21) Komplex-I-Elektronentransportinhibitoren, beispielsweise METI-Akarizide, z.B. Fenazaquin, Fenpyroximate, Pyrimidifen, Pyridaben, Tebufenpyrad und Tolfenpyrad; oder
   Rotenone (Derris).
(22) Spannungsabhängige Natriumkanal-Blocker, z.B. Indoxacarb; oder Metaflumizone.
(23) Inhibitoren der Acetyl-CoA-Carboxylase, wie beispielsweise Tetron- und Tetramsäurederivate, z.B. Spirodiclofen und Spiromesifen.
(24) -IV-Elektronentransportinhibitoren, wie beispielsweise Phosphine, z.B. Aluminiumphosphid, Calciumphosphid, Phosphin und Zinkphosphid; oder
   Cyanid.
(25) Komplex-II-Elektronentransportinhibitoren, wie beispielsweise Cyenopyrafen.
(26) Ryanodinrezeptor-Effektoren, wie beispielsweise Diamide, z.B. Chlorantraniliprole und Flubendiamide.

Weitere Wirkstoffe mit unbekanntem Wirkmechanismus, wie beispielsweise Amidoflumet, Azadirachtin, Benclothiaz, Benzoximate, Bifenazate, Bromopropylate, Chinomethionat, Cryolite, Cyantraniliprole (Cyazypyr), Cyflumetofen, Dicofol, Diflovidazin, Fluensulfone, Flufenerim, Flufiprole, Fluopyram, Fufenozide, Imidaclothiz, Iprodione, Pyridalyl, Pyrifluquinazon und lodmethan; desweiteren Präparate auf Basis von Bacillus firmus (I-1582, BioNeem, Votivo) sowie folgende bekannte wirksame Verbindungen: 3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934), 4-{[(6-Brompyrid-3-yl)methyl](2-fluorethyl)amino}furan-2(5H)-on (bekannt aus WO2007/115644), 4-{[(6-Fluorpyrid-3-yl)methyl](2,2-difluorethyl)amino}furan-2(5H)-on (bekannt aus WO2007/115644), 4-{[(2-Chlor-1,3-thiazol-5-yl)methyl](2-fluorethyl)amino}furan-2(5H)-on (bekannt aus WO2007/115644), 4-{[(6-Chlorpyrid-3-yl)methyl](2-fluorethyl)amino}furan-2(5H)-on (bekannt aus WO2007/115644), 4-{[(6-Chlorpyrid-3-yl)methyl](2,2-difluorethyl)amino}furan-2(5H)-on (bekannt aus WO2007/115644), 4-{[(6-Chlor-5-fluorpyrid-3-yl)methyl](methyl)amino}furan-2(5H)-on (bekannt aus WO2007/115643), 4-{[(5,6-Dichlorpyrid-3-yl)methyl](2-fluorethyl)amino}furan-2(5H)-on (bekannt aus WO2007/115646), 4-{[(6-Chlor-5-fluorpyrid-3-yl)methyl](cyclopropyl)amino}furan-2(5H)-on (bekannt aus WO2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), {[1-(6-Chlorpyridin-3-yl)ethyl](methyl)oxido-λ4-sulfanyliden}cyanamid (bekannt aus WO2007/149134) und seine Diastereomere {[(1R)-1-(6-Chlorpyridin-3-yl)ethyl](methyl)oxido-λ4-sulfanyliden}cyanamid (A) und {[(1S)-1-(6-Chlorpyridin-3-yl)ethyl](methyl)oxido-λ4-sulfanyliden}cyanamid (B) (ebenfalls bekannt aus WO2007/149134) sowie Sulfoxaflor (ebenfalls bekannt aus WO2007/149134) und seine Diastereomere [(R)-Methyl(oxido){(1R)-1-[6-(trifluormethyl)pyridin-3-yl]ethyl}-λ4-sulfanyliden]¬cyanamid (A1) und [(S)-Methyl(oxido){(1S)-1-[6-(trifluormethyl)pyridin-3-yl]ethyl}-λ4-sulfanyliden]cyanamid (A2), bezeichnet als Diastereomerengruppe A (bekannt aus WO 2010/074747, WO 2010/074751), [(R)-Methyl(oxido){(1S)-1-[6-(trifluormethyl)pyridin-3-yl]ethyl}-λ4-sulfanyliden]cyanamid (B1) und [(S)-Methyl(oxido){(1R)-1-[6-(trifluormethyl)pyridin-3-yl]ethyl}-λ4-sulfanyliden]cyanamid (B2), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (bekannt aus WO2006/089633), 3-(4'-Fluor-2,4-dimethylbiphenyl-3-yl)-4-hydroxy-8-oxa-1-azaspiro[4.5]dec-3-en-2-on (bekannt aus WO2008/067911), 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus WO2008/066153), 2-Cyan-3-(difluormethoxy)-N,N-dimethylbenzolsulfonamid (bekannt aus WO2006/056433), 2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407), N-[1-(2,3-Dimethylphenyl)-2-(3,5-dimethylphenyl)ethyl]-4,5-dihydro-1,3-thiazol-2-amin (bekannt aus WO2008/104503), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), (2,2,3,3,4,4,5,5-Octafluorpentyl)(3,3,3-trifluorpropyl)malononitril (bekannt aus WO2005/063094), (2,2,3,3,4,4,5,5-Octafluorpentyl)(3,3,4,4,4-pentafluorbutyl)malononitril (bekannt aus WO2005/063094), 8-[2-(Cyclopropylmethoxy)-4-(trifluormethyl)phenoxy]-3-[6-(trifluormethyl)pyridazin-3-yl]-3-azabicyclo[3.2.1]octan (bekannt aus WO2007/040280), 2-Ethyl-7-methoxy-3-methyl-6-[(2,2,3,3-tetrafluor-2,3-dihydro-1,4-benzodioxin-6-yl)oxy]chinolin-4-yl-methylcarbonat (bekannt aus JP2008/110953), 2-Ethyl-7-methoxy-3-methyl-6-[(2,2,3,3-tetrafluor-2,3-dihydro-1,4-benzodioxin-6-yl)oxy]chinolin-4-ylacetat (bekannt aus JP2008/110953), PF1364 (CAS-Reg.Nr. 1204776-60-2) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (bekannt aus WO2007/075459), 5-[5-(2-Chlorpyridin-4-yl)-5-(trifluormethyl)-4,5-dihydro-1,2-oxazol-3-yl]-2-(1H-1,2,4-triazol-1-yl)benzonitril (bekannt aus WO2007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{[(6-Chlorpyridin-3-yl)methyl](cyclopropyl)amino}-1,3-oxazol-2(5H)-on, 4-{[(6-Chlorpyridin-3-yl)methyl]¬(2,2-difluorethyl)amino}-1,3-oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl](methyl)amino}-1,3-oxazol-2(5H)-on (alle bekannt aus WO2010/005692), NNI-0711 (bekannt aus WO2002/096882), 1-Acetyl-N-[4-(1,1,1,3,3,3-hexafluor-2-methoxypropan-2-yl)-3-isobutylphenyl]-N-isobutyryl-3,5-dimethyl-1H-pyrazol-4-carboxamid (bekannt aus WO2002/096882), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin (bekannt aus WO2007/101369), 2-{6-[2-(5-Fluorpyridin-3-yl)-1,3-thiazol-5-yl]pyridin-2-yl}pyrimidin (bekannt aus WO2010/006713), 2-{6-[2-(Pyridin-3-yl)-1,3-thiazol-5-yl]pyridin-2-yl}pyrimidin (bekannt aus WO2010/006713), 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid (bekannt aus WO2010/069502), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-2H-tetrazol-2-yl]methyl}-1H-pyrazol-5-carboxamid (bekannt aus WO2010/069502) und (1E)-N-[(6-Chlorpyridin-3-yl)methyl]-N'-cyan-N-(2,2-difluorethyl)ethanimidamid (bekannt aus WO2008/009360).

Die hier mit ihrem "common name" genannten Wirkstoffe sind bekannt und beispielsweise im Pestizidhandbuch ("The Pesticide Manual" 16th Ed., British Crop Protection Council 2012) beschrieben oder im Internet recherchierbar (z.B. http://www.alanwood.net/pesticides).

Bei den Pestiziden der Komponente c) kann es sich auch um eine Kombination von zwei oder mehr Pestiziden handeln. Solche Kombinationen sind insbesondere dann von Bedeutung, wenn es beispielsweise darum geht, das Wirkungsspektrum der Pestizid-Zusammensetzung zu verbreitern oder Resistenzen gegenüber bestimmte Pestizide besser zu unterbinden.

In einer bevorzugten Ausführungsform der Erfindung beträgt die Menge des einen oder der mehreren Pestizide der Komponente c) in den erfindungsgemäßen Zusammensetzungen mehr als 100 g/l, bevorzugt mehr als 200 g/l und besonders bevorzugt mehr als 300 g/l. Diese Mengenangaben beziehen sich auf das Gesamtgewicht der erfindungsgemäßen Pestizid-Zusammensetzung und im Falle von Pestiziden, die in Form ihrer wasserlöslichen Salze eingesetzt werden auf die Menge an freier Säure, dem sogenannten Säureäquivalent ("acid equivalent", a.e.).

Der pH-Wert der Pestizidzusammensetzungen liegt üblicherweise im Bereich von 3,5 bis 8,5, bevorzugt bei 4,0 bis 8,0 und besonders bevorzugt bei 4,5 bis 6,5 (gemessen als 1 gew.-%ige wässrige Verdünnung). Der pH-Wert wird primär bestimmt durch die pH-Werte der Lösungen der wässrigen Pestizide, die als Salze schwacher Säuren vorliegen. Durch Zugabe von Säuren oder Basen kann der pH-Wert auf einen anderen Wert abweichend von dem ursprünglichen pH-Wert der Mischung eingestellt werden.

In einer weiteren bevorzugten Ausführungsform der Erfindung liegen die erfindungsgemäßen Pestizidzusammensetzungen als Konzentrat-Formulierungen vor, die vor dem Gebrauch verdünnt werden, insbesondere mit Wasser (beispielsweise "ready-to-use"-, "in-can"- oder "built-in"-Formulierungen), und enthalten das eine oder die mehreren Ammoniumsalze (I) im Allgemeinen in Mengen von 5 bis 80 Gew.-%, bevorzugt von 10 bis 70 Gew.-% und besonders bevorzugt von 20 bis 60 Gew.-%. Diese Mengenangaben beziehen sich auf die gesamte Konzentrat-Formulierung.
Die erfindungsgemäßen Pestizidzusammensetzungen werden vorzugsweise in Form von Spritzbrühen auf die Felder ausgebracht. Dabei werden die Spritzbrühen durch Verdünnung von Konzentrat-Formulierungen mit einer definierten Menge Wasser hergestellt.

In einer weiteren bevorzugten Ausführungsform der Erfindung liegen die erfindungsgemäßen Pestizidzusammensetzungen als Spritzbrühen vor und enthalten 0,001 bis 10 Gew.-%, bevorzugt 0,02 bis 3 Gew.-% und besonders bevorzugt 0,025 bis 2 Gew.-% des einen oder der mehreren Ammoniumsalze (I) Komponente a).

Die Erfindung betrifft weiterhin die Verwendung der erfindungsgemäßen Pestizidzusammensetzungen zur Kontrolle und/oder zur Bekämpfung von Unkraut, Pilzerkrankungen oder Insektenbefall. Bevorzugt ist die Verwendung der erfindungsgemäßen Zusammensetzungen zur Kontrolle und/oder zur Bekämpfung von Unkraut.

Diese Verwendungen können vorzugsweise auch im sogenannten Tank-mix-Verfahren stattfinden. Hierbei können also das oder die mehreren wasserlöslichen Pestizide der Komponente g) und die Komponenten a) bis d) sowie zusätzlich Wasser auch in Form einer sogenannten "Tank-mix"- Zubereitung vorliegen. In einer derartigen Zubereitung liegen sowohl das oder die mehreren wasserlöslichen Pestizide als auch die Komponenten a) bis d), letztere gegebenenfalls zusammen mit weiteren Adjuvants, getrennt voneinander vor. Beide Zubereitungen werden vor dem Ausbringen, in der Regel kurz vorher, miteinander vermischt, wobei eine erfindungsgemäße Pestizid-Zusammensetzung entsteht.

Gegenstand der Erfindung ist auch ein Verfahren zur Bekämpfung von Schadorganismen, wobei man den Schadorganismus oder dessen Lebensraum mit einem erfindungsgemäßen Ammoniumsalz (I) oder einer erfindungsgemäßen Pestizidzusammensetzung in Kontakt bringt. Bei den Schadorganismen handelt es sich vorzugsweise um unerwünschte Pflanzen, wobei das Pestizid dann entsprechend ein Herbizid oder eine Mischung von Herbiziden ist.

### Ausführungsbeispiel

### Herstellung

### Herstellungsbeispiel 1

37 g Dicamba wurden unter Rühren in 30 g deionisiertem Wasser suspendiert. Anschließend wurden unter Rühren 33 g Dimethylglucamin in die Wirkstoffsuspension eingetragen. Es entstand eine klare Lösung.

### Formulierung

Die Zusammensetzung der Wirkstoffformulierung aus dem Herstellungsbeispiel ist in Tabelle 1 zusammengefasst:

**Tabelle 1**

| Zusammensetzung | Beispiel (w%) 1 |
|---|---|
| Dicamba | 37 |
| DMG | 33 |
| Wasser | 30 |
| Aussehen | Klare Lösung |

| | |
|---|---|
| DMG = Dimethylglucamin | |

## Patentansprüche

1. Organisches Ammoniumsalz der Formel (I), 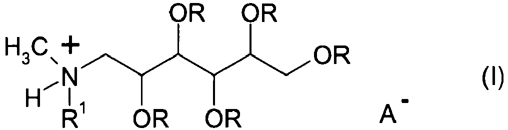 wobei die Symbole folgende Bedeutungen haben:
A⁻ ist ein anionisches Pestizid,
R¹ ist CH₃,
R ist H,
**dadurch gekennzeichnet, dass** A ausgewählt ist aus der Gruppe bestehend aus: Aminocyclopyrachlor, Aminopyralid, Benazolin, Clopyralid, 2,4-D, 2,4-DB, 2,4,5-T, Dicamba, Dichlorprop, Dichlorprop-P, Diflufenzopyr, Fluroxypyr, MCPA, MCPB, Mecoprop, Mecoprop-P, Picloram, Quinclorac, Quinmerac, Tricamba, Triclopyr, Indol-3-essigsäure, 1-Naphthylessigsäure, 2-Naphthylessigsäure und Salicylsäure.

2. Ammoniumsalz gemäß Anspruch 1, **dadurch gekennzeichnet, dass** A ausgewählt ist aus der Gruppe bestehend aus Clopyralide, 2,4-D (2,4-Dichlorphenoxyessigsäure), Dicamba, MCPA, Quinclorac, Quinmerac, Salicylsäure und Triclopyr.

3. Ammoniumsalz gemäß Anspruch 1 oder 2, **dadurch gekennzeichnet, dass** A Dicamba ist.

4. Verfahren zur Herstellung eines Ammoniumsalzes gemäß einem der Ansprüche 1 bis 3, wobei man die protonierte Form eines anionischen Herbizids mit einem Glucamin der Formel (II) umsetzt, 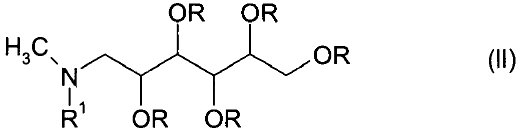 wobei die Symbole die in der Formel (I) angegebenen Bedeutungen haben.

5. Verwendung eines Ammoniumsalzes gemäß einem der Ansprüche 1 bis 4 als Pestizid.

6. Pestizidzusammensetzung, enthaltend
a) ein oder mehrere Ammoniumsalze (I) gemäß einem der Ansprüche 1 bis 3 und
b) einen oder mehrere Formulierungshilfsstoffe.

7. Pestizidzusammensetzung gemäß Anspruch 6 in Form einer Herbizidzusammensetzung.

8. Pestizidzusammensetzung gemäß Anspruch 6 oder 7, enthaltend als Formulierungshilfsstoff b) ein oder mehrere Copolymere A), wobei die Copolymere eine oder mehrere Struktureinheiten, hervorgegangen aus
a) 19,9 bis 75,9 Gew.-% Glycerin
b) 0,1 bis 30 Gew.-% mindestens einer Dicarbonsäure und
c) 24 bis 80 Gew.-% mindestens einer Monocarbonsäure gemäß Formel (III),
R²-COOH (III)
wobei R² (C₅-C₂₉)-Alkyl; (C₇-C₂₉)-Alkenyl; Phenyl oder Naphthyl darstellt, enthalten.

9. Verfahren zur Bekämpfung von Schadorganismen, wobei man den Schadorganismus oder dessen Lebensraum mit einem Ammoniumsalz (I) gemäß einem der Ansprüche 1 bis 3 oder einer Pestizidzusammensetzung gemäß einem der Ansprüche 6 bis 8 in Kontakt bringt.

10. Verfahren gemäß Anspruch 9, wobei es sich bei den Schadorganismen um unerwünschte Pflanzen und bei dem Pestizid um ein Herbizid handelt.

11. Verwendung eines Ammoniumsalzes gemäß einem der Ansprüche 1 bis 3 zur Verminderung der Flüchtigkeit des als Salz vorliegenden anionischen Pestizids.

## Claims

1. Organic ammonium salt of the formula (I) 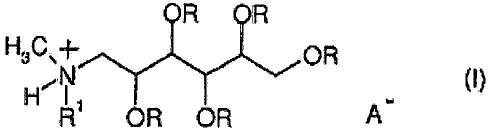 where the symbols have the following definitions:
A⁻ is an anionic pesticide,
R¹ is CH₃,
R is H,
**characterized in that** A is selected from the group consisting of: aminocyclopyrachlor, aminopyralid, benazolin, clopyralid, 2,4-D, 2,4-DB, 2,4,5-T, dicamba, dichlorprop, dichlorprop-P, diflufenzopyr, fluroxypyr, MCPA, MCPB, mecoprop, mecoprop-P, picloram, quinclorac, quinmerac, tricamba, triclopyr, indole-3-acetic acid, 1-naphthylacetic acid, 2-naphthylacetic acid and salicylic acid.

2. Ammonium salt according to Claim 1, **characterized in that** A is selected from the group consisting of clopyralid, 2,4-D (2,4-dichlorophenoxyacetic acid), dicamba, MCPA, quinclorac, quinmerac, salicylic acid and triclopyr.

3. Ammonium salt according to Claim 1 or 2, **characterized in that** A is dicamba.

4. Process for preparing an ammonium salt according to any of Claims 1 to 3, wherein the protonated form of an anionic herbicide is reacted with a glucamine of the formula (II) 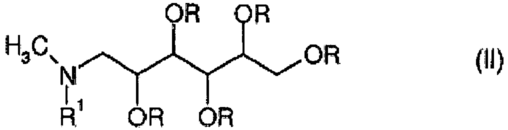 where the symbols have the definitions given in the formula (I).

5. Use of an ammonium salt according to any of Claims 1 to 4 as a pesticide.

6. Pesticide composition comprising
a) one or more ammonium salts (I) according to any of Claims 1 to 3 and
b) one or more formulation auxiliaries.

7. Pesticide composition according to Claim 6 in the form of a herbicide composition.

8. Pesticide composition according to Claim 6 or 7, comprising, as formulation auxiliary b), one or more copolymers A), where the copolymers contain one or more structural units derived from
a) 19.9% to 75.9% by weight of glycerol
b) 0.1% to 30% by weight of at least one dicarboxylic acid and
c) 24% to 80% by weight of at least one monocarboxylic acid of formula (III)
R²-COOH (III)
where R² is (C₅-C₂₉)-alkyl; (C₇-C₂₉)-alkenyl; phenyl or naphthyl.

9. Method of controlling harmful organisms, wherein the harmful organism or its habitat is brought into contact with an ammonium salt (I) according to any of Claims 1 to 3 or a pesticide composition according to any of Claims 6 to 8.

10. Method according to Claim 9, wherein the harmful organisms are unwanted plants and the pesticide is a herbicide.

11. Use of an ammonium salt according to any of Claims 1 to 3 for reducing the volatility of the anionic pesticide present in salt form.

## Revendications

1. Sel d'ammonium organique de formule (I) 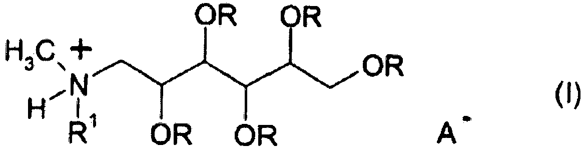 dans laquelle les symboles ont les significations suivantes :
A⁻ est un pesticide anionique,
R¹ représente CH₃,
R représente H,
**caractérisé en ce qu'**A est choisi dans le groupe constitué par : l'aminocyclopyrachlor, l'aminopyralide, la bénazoline, le clopyralide, le 2,4-D, le 2,4-DB, le 2,4,5-T, le dicamba, le dichlorprop, le dichlorprop-P, le diflufenzopyr, le fluroxypyr, le MCPA, le MCPB, le mécoprop, le mécoprop-P, le picloram, le quinclorac, le quinmérac, le tricamba, le triclopyr, l'acide indol-3-acétique, l'acide 1-naphtylacétique, l'acide 2-naphtylacétique et l'acide salicylique.

2. Sel d'ammonium selon la revendication 1, **caractérisé en ce qu'**A est choisi dans le groupe constitué par le clopyralide, le 2,4-D, (acide 2,4-dichlorophénoxyacétique), le dicamba, le MCPA, le quinclorac, le quinmérac, l'acide salicylique et le triclopyr.

3. Sel d'ammonium selon la revendication 1 ou 2, **caractérisé en ce qu'**A est le dicamba.

4. Procédé de fabrication d'un sel d'ammonium selon l'une quelconque des revendications 1 à 3, dans lequel la forme protonée d'un herbicide anionique est mise en réaction avec une glucamine de formule (II) 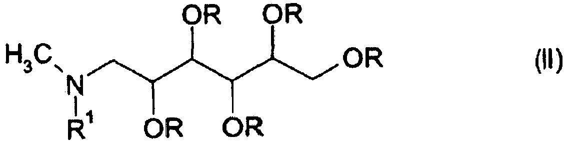 dans laquelle les symboles ont les significations indiquées dans la formule (I).

5. Utilisation d'un sel d'ammonium selon l'une quelconque des revendications 1 à 4 en tant que pesticide.

6. Composition pesticide, contenant :
a) un ou plusieurs sels d'ammonium (I) selon l'une quelconque des revendications 1 à 3, et
b) un ou plusieurs adjuvants de formulation.

7. Composition pesticide selon la revendication 6, sous la forme d'une composition herbicide.

8. Composition pesticide selon la revendication 6 ou 7, contenant en tant qu'adjuvants de formulation b) un ou plusieurs copolymères A), les copolymères contenant une ou plusieurs unités structurales, dérivées de :
a) 19,9 à 75,9 % en poids de glycérine,
b) 0,1 à 30 % en poids d'au moins un acide dicarboxylique et
c) 24 à 80 % en poids d'au moins un acide monocarboxylique selon la formule (III)
R²-COOH (III)
dans laquelle R² représente alkyle en (C₅-C₂₉), alcényle en (C₇-C₂₉), phényle ou naphtyle.

9. Procédé de lutte contre des organismes nuisibles, selon lequel l'organisme nuisible ou son habitat est mis en contact avec un sel d'ammonium (I) selon l'une quelconque des revendications 1 à 3 ou une composition pesticide selon l'une quelconque des revendications 6 à 8.

10. Procédé selon la revendication 9, dans lequel les organismes nuisibles sont des plantes indésirables et le pesticide est un herbicide.

11. Utilisation d'un sel d'ammonium selon l'une quelconque des revendications 1 à 3 pour réduire la volatilité du pesticide anionique présent sous la forme d'un sel.
